# FASCICULE DE BREVET EUROPEEN

(11) **EP 3 013 248 B1**
(45) Date de publication et mention de la délivrance du brevet: **16.08.2017**
(21) Numéro de dépôt: 14735546.5
(22) Date de dépôt: 30.06.2014
(51) Int. Cl.: A61B 10/02

(54) **DISPOSITIF DE PRELEVEMENT IN VIVO D'ESPECES BIOLOGIQUES**
VORRICHTUNG ZUR IN-VIVO ENTNAHME BIOLOGISCHER PROBEN
DEVICE FOR IN VIVO SAMPLING OF BIOLOGICAL SPECIMEN

(30) Priorité: 28.06.2013 FR 1356289
(43) Date de publication de la demande: 04.05.2016
(73) Titulaire: Commissariat à l'Énergie Atomique et aux Énergies Alternatives, 75015 Paris (FR)
(72) Inventeur: BOUAMRANI, Mohamed-Ali, F-38000 Grenoble (FR); BERGER, François, F-38240 Meylan (FR); DREYFUS, Matthieu, F-38000 Grenoble (FR); MOMBRUN, Adrien, F-38000 Grenoble (FR)
(74) Mandataire: Regimbeau
(86) Numéro de dépôt international: PCT/EP2014/063806
(87) Numéro de publication internationale: WO 2014/207254

(56) Documents cités:
- EP-A2- 0 277 009
- WO-A1-01/97693
- WO-A1-2011/090778
- US-A1- 2005 010 095
- US-A1- 2009 317 835
- US-A1- 2013 079 663

## Description

### DOMAINE DE L'INVENTION

La présente invention concerne un dispositif de prélèvement in vivo d'espèces biologiques.

### ARRIERE PLAN DE L'INVENTION

L'analyse tissulaire est le meilleur indicateur pour accéder aux informations biologiques pertinentes provenant directement d'un foyer pathologique.

L'exploration micro-invasive repose principalement sur la ponction-biopsie, qui peut être réalisée sous une voie d'abord endoscopique ou coelioscopique.

De nombreux dispositifs miniaturisés ont ainsi été développés pour atteindre les organes profonds et le tissu cible en passant de manière atraumatique par les voies naturelles, comme le tube digestif, le système cardiovasculaire, l'appareil urinaire ou l'appareil respiratoire. Les documents US 2009/317835, WO 01/97693, et US 2013/079663 décrivent de tels dispositifs, permettant de réaliser des prélèvements in vivo d'espèces biologiques au moyen d'un support de capture adapté.

Ces biopsies entraînent cependant un risque hémorragique ou infectieux qui n'est pas négligeable du fait des lésions tissulaires occasionnées.

De ce fait, de nombreux territoires pathologiques, tels que la région péri-tumorale laissée en place par le chirurgien restent donc inaccessibles.

De plus, l'important polymorphisme mutationnel requiert un échantillonnage extensif de la tumeur, incompatible avec les approches biopsiques micro-invasives.

D'autre part, en raison de leur coût et du temps requis pour leur mise en oeuvre, les procédures actuelles sont difficilement compatibles avec le besoin de disposer d'analyses extemporanées, c'est-à-dire réalisées au bloc opératoire pendant une intervention chirurgicale, afin d'aider les décisions du chirurgien.

L'invention a pour objectif de remédier aux inconvénients des dispositifs existants et de permettre un recueil de molécules pathologiques mais aussi de cellules qui n'occasionne pas de lésion des tissus et qui soit en outre compatible avec des analyses extemporanées.

### BREVE DESCRIPTION DE L'INVENTION

Conformément à l'invention, il est proposé un dispositif de prélèvement in vivo d'espèces biologiques, comprenant :
- un support de capture en un matériau nanoporeux, présentant une surface de capture desdites espèces, ledit support de capture étant porté par un plateau,
- une tige de préhension,
- une articulation comprenant un premier membre couplé à la tige de préhension et un second membre couplé de manière amovible au plateau portant le support de capture, de sorte que la surface de capture est sélectivement orientable par rapport à la tige de préhension, de façon à ajuster l'orientation de la surface de capture par rapport à la tige de préhension.

On entend par « matériau nanoporeux » un matériau cristallin ou amorphe, d'un seul tenant et de préférence de composition homogène, présentant des pores dont le diamètre moyen est inférieur à un micromètre et en particulier inférieur ou égal à 100 nm. On distingue notamment, parmi les matériaux nanoporeux, les matériaux microporeux, (pores de diamètre moyen compris entre 0,2 et 2 nm), les matériaux mésoporeux (pores de diamètre moyen compris entre 2 et 50 nm) et les matériaux macroporeux (pores de diamètre moyen compris entre 50 et 1000 nm).

De préférence, la porosité dudit matériau, à savoir le rapport entre le volume des pores par rapport au volume total du matériau, est supérieure ou égale à 10%.

Les espèces biologiques susceptibles d'être adsorbées sur ledit matériau nanoporeux peuvent être des cellules (dont le diamètre est compris entre 1 µm et 50 µm environ), des bactéries, des virus, des vésicules circulantes comme les exosomes (diamètre compris entre 20 et 200 nm environ), mais aussi des molécules ou des macromolécules, telles que des protéines (diamètre compris entre quelques nanomètres et quelques dizaines de nanomètres), des peptides (taille de l'ordre du nanomètre), des métabolites. La « taille » de ces molécules s'entend comme leur plus grande dimension.

Le dispositif selon l'invention permet de s'affranchir du prélèvement d'un fragment de tissu en capturant des espèces biologiques par simple contact entre la surface de capture nanoporeuse et le tissu d'intérêt.

Ledit dispositif permet donc de réaliser une empreinte moléculaire et cellulaire s'intégrant aux explorations endoscopiques ou coelioscopiques, notamment lors de l'abord exploratoire de tissus ou d'organes non biopsiables.

En effet, la surface de capture nanoporeuse a la capacité, au contact du tissu vivant, de capturer une couche de cellules superficielles, de manière non lésionnelle, tout en conservant la structure histologique du tissu ciblé. Ladite surface permet en outre de retenir les espèces moléculaires présentes dans le liquide interstitiel du tissu.

D'autre part, le matériel biologique capturé sur la surface nanoporeuse peut être directement analysé au moyen de techniques histologiques classiques (coloration, immuno marquage), mais aussi par spectrométrie de masse MALDI (imagerie et/ou profilage). Par « directement », on entend que l'analyse est effectuée directement sur le matériel biologique présent sur le support de capture, sans le séparer dudit support.

De manière particulièrement avantageuse, le premier et le second membres de l'articulation sont séparables de sorte à détacher le support de capture de la tige de préhension.

De préférence, le support de capture est encastré dans le plateau, ledit plateau présentant un rebord affleurant avec la surface de capture, de sorte que seule la surface de capture dudit support soit en contact avec des tissus lors du prélèvement in vivo.

De préférence, le plateau est en un matériau électriquement conducteur, ce qui permet de le conserver solidaire du support de capture pour des analyses de type MALDI.

Selon un mode de réalisation, l'articulation est une rotule.

Selon une forme d'exécution de l'invention, le matériau du support de capture est du silicium nanoporeux.

De manière avantageuse, la surface de capture est inscrite dans un cercle de moins de 10 mm de diamètre.

Le dispositif peut en outre comprendre un dispositif d'orientation du support de capture, comprenant un câble s'étendant le long de la tige de préhension et couplé au second membre de l'articulation.

Un tel dispositif permet avantageusement la mise en oeuvre d'un procédé automatisé d'analyse d'espèces biologiques capturées au moyen d'un tel dispositif, comprenant :
- la fourniture d'une pluralité de supports de capture comprenant des espèces biologiques adsorbées sur la surface de capture de chaque support,
- la fourniture d'un support de mesure présentant la forme d'une plaque électriquement conductrice comprenant une pluralité de logements dont la forme et les dimensions sont adaptées à celles des supports de capture pour permettre un contact électrique entre le support de mesure et chaque support de capture,
- la mise en place de chaque support de capture dans un logement correspondant du support de mesure,
- le dépôt automatisé d'une matrice organique sur chaque surface de capture,
- l'acquisition automatisée de mesures de spectrométrie de masse sur chaque surface de capture.

Selon une forme d'exécution de l'invention, le plateau dans lequel est encastré le support de capture est électriquement conducteur, ledit plateau présentant un rebord affleurant avec la surface de capture, et l'on met en place, dans chaque logement du support de mesure, le support de capture et le plateau dans lequel il est encastré.

Selon un mode de réalisation particulier, le support de mesure comprend 96 logements pour des supports de capture.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma de principe d'un dispositif de prélèvement selon un mode de réalisation de l'invention,
- les figures 2A à 2D illustrent de manière schématique différentes étapes de l'utilisation d'un tel dispositif de prélèvement,
- la figure 3A est une vue en perspective d'un support de mesure pour l'analyse automatisée d'une pluralité de supports de capture ; la figure 3B est une vue en coupe d'un logement d'un tel support dans lequel sont agencés un support de capture et le plateau dans lequel il est encastré,
- la figure 4 montre des spectres de masse de protéines et de peptides du liquide céphalo-rachidien humain capturés au moyen d'un support de capture en silicium nanoporeux et d'un support de référence lisse,
- la figure 5 montre des spectres de masse obtenus pour deux zones d'une même prostate résultant d'une exérèse.

### DESCRIPTION DETAILLEE DE L'INVENTION

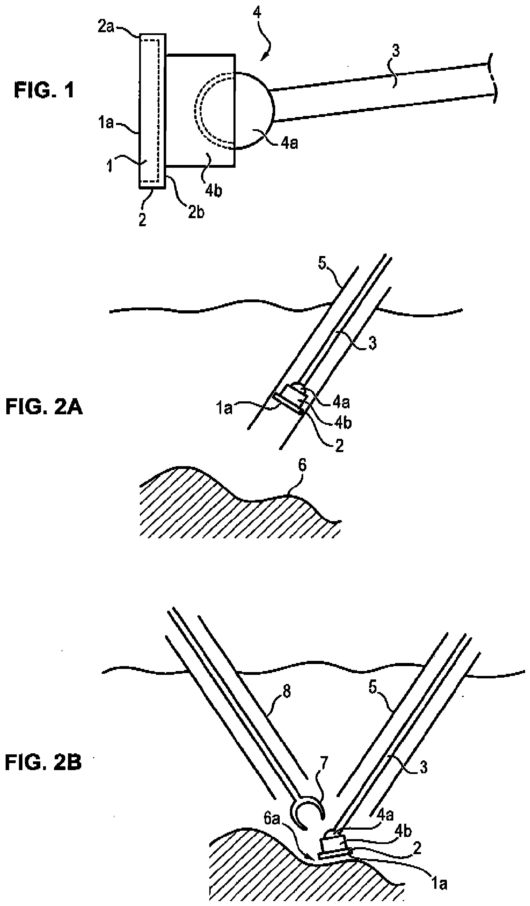

La figure 1 illustre de manière schématique un dispositif de prélèvement selon un mode de réalisation de l'invention.

Ledit dispositif comprend un support de capture 1 dont une surface est destinée à former la surface de capture 1a des espèces biologiques à prélever.

La surface de capture 1a peut présenter une forme rectangulaire, circulaire ou toute autre forme.

La surface de capture 1a est généralement plane.

De préférence, la surface de capture 1a est inscrite dans un cercle de moins de 10 mm de diamètre, de sorte à pouvoir être insérée, selon les cas, dans un trocart de coelioscopie ou un canal opérateur d'endoscopie conventionnel pour être amenée jusqu'aux tissus dans lesquels on souhaite effectuer un prélèvement.

La plus grande dimension de la surface de capture 1a est généralement supérieure à l'épaisseur du support 1.

A titre purement indicatif, la surface de capture peut être un carré de 5 mm de côté, tandis que l'épaisseur du support de capture peut être comprise entre 100 µm et 2 mm, par exemple de l'ordre de 750 µm.

Le support de capture 1 est en un matériau nanoporeux.

Selon un mode de réalisation préféré, le matériau nanoporeux est du silicium nanoporeux.

Avantageusement, ledit silicium nanoporeux peut présenter au moins l'une des propriétés suivantes, et préférentiellement l'ensemble de ces propriétés :
- des pores de structure dendritique,
- des pores de diamètre moyen compris entre 1 et 100 nm,
- une porosité comprise entre 40% et 65% sur une profondeur comprise entre 10 nm et 100 µm.

Un avantage du silicium nanoporeux est qu'il ne nécessite pas de fonctionnaliser la surface de capture pour adsorber des espèces biologiques.

Un autre avantage du silicium nanoporeux est qu'il supporte les traitements de stérilisation classiquement appliqués aux instruments chirurgicaux, en particulier par autoclave.

Du silicium nanoporeux (et plus particulièrement mésoporeux) peut être obtenu par anodisation électrochimique de silicium dopé p+ de conductivité de 10 à 20 mΩ.cm dans une solution d'acide fluorhydrique (HF) à environ 15%. Pour ce faire, le matériau est trempé dans un bain d'HF et soumis à une électrolyse, ce procédé étant en lui-même connu. On obtient de cette façon un matériau présentant une porosité de structure dendritique ; cela signifie que les pores n'ont pas un axe rectiligne et qu'ils s'étendent dans la profondeur du matériau selon une direction discontinue et peuvent se croiser. Cette structure dendritique favorise la succion. La porosité est comprise entre 40 et 65%. La porosité s'étend sur une profondeur d'environ 6 µm ; au-delà, on retrouve le silicium massif. D'une façon générale, pour un support de capture utilisable dans l'invention, la porosité du matériau s'étend sur une profondeur pouvant être comprise entre 10 nm et 100 µm.

Naturellement, c'est une surface poreuse dudit matériau qui est employée comme surface de capture.

En faisant varier les paramètres de fabrication (concentration en HF, temps d'anodisation, densité de courant, type de silicium), on peut faire varier les caractéristiques du silicium nanoporeux.

En variante, il est possible de fabriquer du silicium nanoporeux présentant une structure ordonnée au moyen d'un procédé de lithographie électronique.

Naturellement, tout autre procédé d'obtention de silicium nanoporeux pourrait être utilisé sans pour autant sortir du cadre de la présente invention.

D'autre part, l'invention n'est pas limitée au silicium nanoporeux et l'homme du métier pourrait également choisir un autre matériau nanoporeux, par exemple un copolymère block, un matériau de type sol-gel, un matériau de type SiOCH rendu poreux. De tels matériaux sont notamment rendus poreux par le recours à des agents porogènes, selon des procédés connus.

De manière avantageuse, le support de capture 1 est porté par un plateau 2.

De préférence, ledit plateau 2 présente un logement pour le support, ledit logement étant délimité par un rebord 2a dont la hauteur est sensiblement égale à l'épaisseur du support 1.

Ainsi, la surface de capture 1a affleure avec le rebord 2a du plateau, de sorte que seule la surface de capture 1a est en contact avec les tissus lors du prélèvement.

On évite ainsi que les bords du support de capture, qui sont susceptibles d'être coupants, n'endommagent les tissus.

Par ailleurs, les formes extérieures du plateau 2 sont conçues de sorte à ne pas être agressives pour les tissus ; en particulier, elles ne présentent de préférence pas d'angle vif.

L'encombrement extérieur du plateau 2 est de préférence aussi réduit que possible, de sorte à faciliter son insertion jusqu'au site de prélèvement.

Le plateau est avantageusement en métal ; comme on le verra plus bas, le fait que le plateau soit électriquement conducteur permet de conserver le support de capture 1 dans le plateau 2 pour des analyses de spectrométrie de masse.

Cependant, ce mode de réalisation n'est pas limitatif et l'on peut choisir pour le plateau 2 tout autre matériau approprié susceptible d'être stérilisé.

Le support de capture 1 est maintenu dans son logement par tout moyen, amovible ou non.

Ledit moyen peut comprendre de la colle, notamment une colle biocompatible, un encliquetage, etc.

De manière avantageuse, le plateau peut présenter une forme parallélépipédique ou cylindrique, ce qui facilite son utilisation lorsqu'il est destiné à maintenir le support de capture lors d'analyses ultérieures.

En particulier, si le plateau 2 présente un fond 2b plat, il peut être posé de manière stable sur une table et mis en contact avec un support de mesure plan.

Le dispositif de prélèvement comprend en outre une tige de préhension 3 permettant la manipulation du support de capture 1 lors de son introduction dans le corps et du prélèvement sur les tissus d'intérêt.

Si les tissus sont abordés par coelioscopie, la tige 3 est rigide, ce qui facilite son insertion au travers d'un trocart.

Si les tissus sont abordés par endoscopie, la tige 3 est flexible, de sorte à pouvoir emprunter des canaux opérateurs.

L'homme du métier est en mesure de sélectionner le matériau approprié pour la tige 3 selon l'application prévue, en tenant compte des contraintes liées à la stérilisation du dispositif.

Le diamètre de la tige est adapté pour le passage au travers d'un trocart ou d'un canal opérateur d'endoscopie.

La longueur de la tige 3 est variable selon le mode de manipulation envisagé.

Si la tige est destinée à être manipulée depuis l'extérieur du corps, sa longueur est supérieure à la distance entre les tissus d'intérêt et l'extérieur du corps.

La tige peut alors présenter une longueur de quelques dizaines de centimètres.

Eventuellement, la tige peut être télescopique.

La tige peut en revanche être plus courte si elle est portée par une pince ou un robot jusqu'au site de prélèvement ; dans ce cas, une longueur de quelques centimètres est suffisante.

La surface de capture 1a est orientable par rapport à la tige 3 par l'intermédiaire d'une articulation 4.

De préférence, ladite articulation est une rotule, car elle autorise le plus grand nombre de degrés de liberté pour l'inclinaison de la surface de capture.

II est cependant possible de choisir une autre articulation, par exemple une charnière.

L'articulation 4 comprend un premier membre 4a couplé à la tige 3 et un second membre 4b couplé au plateau portant le support de capture, le premier et le second membre étant orientables l'un par rapport à l'autre.

Si l'articulation est une rotule, le premier membre est une bille et le second membre est une coupelle présentant un logement sphérique pour la bille, ou inversement.

De manière optionnelle, les premier et second membres sont séparables l'un de l'autre, par exemple en leur conférant une élasticité suffisante pour permettre une ou plusieurs séquences d'encliquetage et de décliquetage.

L'homme du métier choisit un matériau approprié pour les deux membres de l'articulation, en tenant compte notamment des contraintes de stérilisation et éventuellement de démontage.

Le premier membre 4a est couplé à la tige 3 par tout moyen approprié, amovible ou non.

Ledit premier membre 4a est par exemple collé à l'extrémité de la tige 3, notamment au moyen d'une colle biocompatible.

De manière alternative, le premier membre 4a peut faire partie intégrante de la tige 3, c'est-à-dire fabriqué en une seule pièce avec celle-ci.

Le second membre 4b de l'articulation est couplé de manière amovible au plateau 2.

La liaison entre le plateau 2 et le second membre 4b est ainsi démontable, par exemple par encliquetage, vissage, etc.

Dans ce cas, il n'est pas nécessaire que l'articulation 4 elle-même soit démontable.

Le fait de pouvoir séparer le plateau 2 portant le support de capture 1 de la tige 3 (soit en désolidarisant les deux membres 4a, 4b de l'articulation, soit en désolidarisant le plateau 2 du second membre 4b de l'articulation) permet de remplacer un support déjà utilisé par un nouveau support, porté par un nouveau plateau.

On peut ainsi réaliser successivement des prélèvements sur un même site en utilisant la même tige et en y attachant successivement plusieurs supports de capture.

La conception permettant de désolidariser le plateau 2 de l'ensemble constitué de la tige 3 et des deux membres 4a, 4b de l'articulation est préférable car elle permet de conserver le mécanisme le plus complexe pour le réutiliser et de remplacer simplement le plateau, dont la géométrie est généralement plus simple et qui est moins onéreux.

De préférence, on choisit une liaison entre le plateau 2 et le second membre 4b de l'articulation qui soit aisée et rapide à démonter, de préférence sans nécessiter d'outils.

Naturellement, pour des raisons de sécurité, toute liaison démontable dans le dispositif de prélèvement doit être suffisamment forte pour éviter toute désolidarisation intempestive d'un élément vis-à-vis du reste du dispositif, notamment lorsque celui-ci est à l'intérieur du corps.

De préférence, les liaisons démontables sont réalisées par un encliquetage suffisamment rigide pour être convenablement solidarisé au reste du dispositif.

Selon un mode de réalisation (non illustré), la surface de capture 1a est orientable par un utilisateur depuis l'extérieur.

Le dispositif de prélèvement comprend à cet effet un dispositif d'orientation connecté au second membre de l'articulation et actionnable de l'extérieur.

Par exemple, ledit dispositif d'orientation comprend un câble fixé au second membre de l'articulation et s'étendant le long de la tige de préhension 3.

On va maintenant décrire un mode opératoire du dispositif de prélèvement, en référence aux figures 2A à 2D.

D'une manière générale, le dispositif peut être utilisé pour prélever des espèces biologiques dans tout organe ou tissu abordé par endoscopie ou coelioscopie.

Par exemple, et de manière non limitative, ledit dispositif peut être utilisé pour des prélèvements dans la prostate, dans la vessie, dans l'estomac, etc.

Une fois le support de capture connecté à l'extrémité de la tige de préhension par l'intermédiaire de l'articulation, le dispositif est introduit dans un trocart ou dans un canal opérateur d'endoscopie 5 (figure 2A).

Si la tige de préhension n'est pas suffisamment longue pour être manipulée directement par l'utilisateur depuis l'extérieur, elle est tenue par une pince actionnée manuellement ou par un robot (non illustrée).

Le trocart ou le canal opérateur 5 protège la surface de capture 1a au cours de son insertion puis de son retrait.

II n'est donc pas nécessaire de munir le dispositif de prélèvement d'une protection spécifique de la surface de capture.

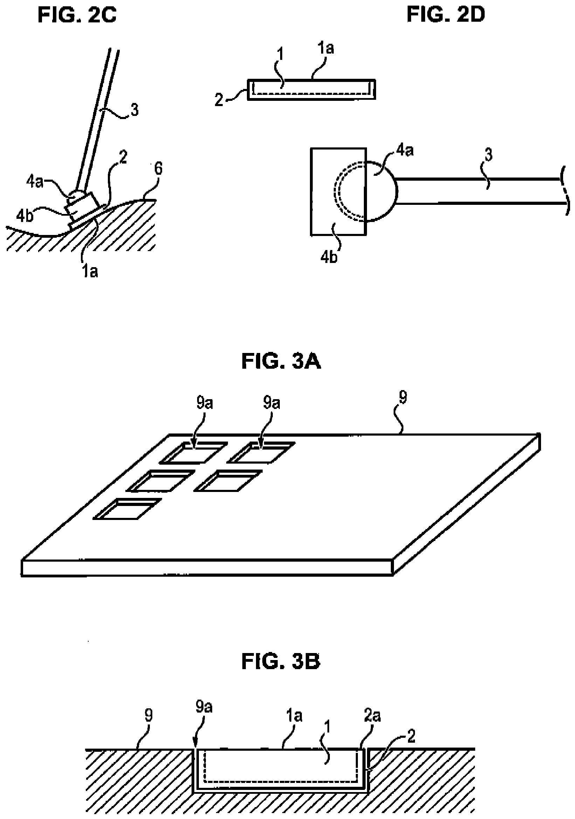

Lorsque le support de capture 1 se trouve au niveau du tissu 6 dans lequel on souhaite faire des prélèvements, on ajuste l'orientation de la surface de capture 1a de sorte à la rendre sensiblement parallèle à la surface 6a du tissu dans la zone de prélèvement (figure 2B), puis on applique la surface de capture contre le tissu, à la manière d'un tampon (figure 2C).

II n'est pas nécessaire à cet effet d'exercer une pression sur le support de capture, un contact étant suffisant pour permettre la capture des espèces biologiques.

La surface de capture en matériau nanoporeux est lisse au toucher et ne présente pas d'aspérité significative, ce qui minimise les risques de lésion des tissus.

Le prélèvement des espèces biologiques n'est donc pas réalisé par micro-abrasion ni par fonctionnalisation chimique mais par un effet de succion dû aux nanopores.

Cet effet conduit à une fixation préférentielle des peptides et des « petites » molécules, présentant une taille de l'ordre de 1 à 5 nm environ et/ou une masse comprise entre 200 et 20000 Da environ.

Cette propriété est avantageuse car ces petites molécules sont généralement plus utiles en tant que marqueurs que des molécules de plus grandes dimensions.

L'effet de succion explique également l'adhésion des cellules, qui sont trop grandes pour pénétrer dans les pores.

En termes d'usage, le maniement du dispositif est différent d'un dispositif de biopsie tel que décrit dans le document WO 2006/082344, dans lequel la surface de capture est agencée sur une portion de la tige en s'étendant dans un plan parallèle à l'axe longitudinal de la tige.

En effet, dans ce dispositif connu, le geste de l'utilisateur consiste à manipuler la tige tangentiellement au tissu dans lequel on souhaite effectuer un prélèvement, de sorte à y apposer la surface de capture.

Cette nécessité d'une approche tangentielle de la tige peut poser des problèmes d'accessibilité vis-à-vis de certains organes, la longueur de la tige étant susceptible d'empêcher l'accès à des régions de petites dimensions.

Au contraire, avec un dispositif orientable selon l'invention, l'utilisateur manipule la tige dans une direction qui n'est pas nécessairement tangentielle au tissu mais qui peut être inclinée par rapport à celui-ci, en orientant la surface de capture de sorte à ce qu'elle vienne en contact du tissu parallèlement à celui-ci.

Grâce à cette faculté d'orienter sélectivement uniquement le support de capture 1a et le second membre 4b de l'articulation, qui présentent des dimensions réduites, par rapport à la tige 3, il est possible d'accéder plus facilement aux tissus d'intérêt, même lorsque la zone de prélèvement est de petites dimensions. Par « sélectivement », on entend que l'on peut ajuster l'orientation de la surface de capture par rapport à la tige de préhension.

Comme indiqué plus haut, l'orientation de la surface de capture 1a par rapport à la tige peut être réalisée par un dispositif d'orientation incorporé au dispositif de prélèvement.

Par exemple, s'il s'agit d'un câble, une traction de l'utilisateur permet de faire varier l'inclinaison du second membre de l'articulation par rapport au premier.

Cependant, le dispositif de prélèvement peut également être passif, c'est-à-dire ne pas comprendre lui-même de moyen permettant d'ajuster l'orientation de la surface de capture depuis l'extérieur.

Dans ce cas, et comme illustré sur la figure 2B, l'orientation de la surface de capture 1a est réalisée en manipulant le second membre 4b de l'articulation au moyen d'une pince 7 introduite au niveau du site de prélèvement par un autre trocart ou canal opérateur 8, ladite pince 7 étant manipulée par l'autre main de l'utilisateur, par un autre utilisateur ou par un robot, jusqu'à ce que la surface de capture 1a soit orientée de manière adéquate par rapport au tissu 6.

Une fois que le prélèvement a été effectué, l'utilisateur retire le dispositif de prélèvement par le trocart ou le canal opérateur par lequel il a été introduit.

En vue d'analyser les espèces capturées, on sépare le support de capture 1a (éventuellement solidaire du plateau 2 et/ou du second membre de l'articulation) du reste du dispositif de prélèvement (cf. figure 2D).

Si d'autres prélèvements sont à effectuer, il suffit de remettre en place un support de capture vierge (éventuellement solidaire d'un nouveau plateau et/ou d'un nouveau second membre de l'articulation) pour un nouveau prélèvement.

Un rinçage du support de capture permet de retirer les espèces qui n'auraient pas été adsorbées sur la surface de capture et/ou des impuretés qui se seraient déposées sur celle-ci.

On analyse ensuite les espèces biologiques capturées dans le support de capture.

De préférence, cette analyse est effectuée de manière extemporanée, c'est-à-dire au bloc opératoire, afin d'éclairer rapidement le chirurgien sur la poursuite de l'intervention chirurgicale.

Par exemple, dans le cas de la prostatectomie, le chirurgien effectue des empreintes tissulaires au niveau de la région qu'il a déjà retirée, et le résultat des analyses lui permet de déterminer s'il subsiste des tissus cancéreux à retirer ou s'il peut cesser l'intervention.

Ceci permet d'éviter de retirer plus de tissus que nécessaire et minimise ainsi les risques de séquelles (incontinence, impuissance) pour le patient.

De manière particulièrement avantageuse, le support de capture peut être utilisé directement pour réaliser ces analyses, sans qu'il soit nécessaire de désorber les espèces du matériau nanoporeux pour les en séparer.

Ainsi, le support de capture, éventuellement solidaire du plateau, peut être introduit dans un appareillage de mesure adapté pour analyser les espèces capturées.

Typiquement, les analyses peuvent être effectuées par spectrométrie de masse avec désorption laser, de type MALDI ou SELDI.

Le profil protéique obtenu est en effet spécifique de la nature pathologique ou non du tissu étudié et constitue un complément à l'analyse histologique clinique.

Pour la mise en oeuvre de la spectrométrie de masse directement sur le support de capture, le matériau nanoporeux doit être de préférence électriquement conducteur, ce qui est le cas du silicium nanoporeux.

De manière alternative, si le matériau nanoporeux n'est pas électriquement conducteur, le support de capture doit être suffisamment fin pour permettre une analyse de type MALDI ou SELDI. Dans un tel cas, il est déposé sur un matériau électriquement conducteur.

De manière avantageuse, si le plateau est également électriquement conducteur, le support de capture peut être maintenu dans le plateau et l'ensemble est introduit dans le spectromètre de masse.

Au préalable, on dépose sur la surface de capture une matrice organique appropriée qui co-cristallise avec les espèces adsorbées et permet ensuite leur désorption par le laser.

Cette technique étant connue de l'homme du métier, elle ne sera pas décrite en détail ici.

Selon un mode de réalisation particulièrement avantageux, il est possible d'analyser de manière automatisée une pluralité de supports de capture.

A cet effet, comme illustré sur la figure 3A, l'invention propose un support de mesure 9 dont les dimensions sont adaptées pour son introduction dans le spectromètre de masse et qui présente un logement 9a ou une pluralité de logements 9a adaptés pour recevoir les supports de capture (éventuellement encastrés dans les plateaux précités).

Le support de mesure présente avantageusement la forme d'une plaque et est réalisé en un matériau électriquement conducteur.

Chaque logement 9a présente une forme et des dimensions sensiblement identiques à celles de la surface de capture 1a (ou, le cas échéant, de la forme extérieure du plateau 2), de sorte que le support de capture 1 et, le cas échéant le plateau 2, soit encastrés dans le support de mesure, de sorte à assurer un contact électrique entre le support de mesure et le support de capture.

La figure 3B est une vue en coupe du support de mesure 9 dans lequel un support de capture 1 et le plateau dans lequel il est encastré sont reçus dans un logement 9a.

De préférence, la surface de capture 1a est affleurante avec la surface du support de mesure 9.

De manière particulièrement avantageuse, le support de mesure comprend 96 logements agencés de sorte à rendre ledit support de mesure compatible avec les dispositifs automatisés existants de traitement des échantillons (notamment de dépôt de la matrice organique) et d'analyse.

En variante ou en complément, on peut effectuer des analyses par imagerie, notamment imagerie de fluorescence ou colorimétrie.

II est également possible d'acquérir des images de microscopie électronique en balayage de la surface de capture.

II est également possible d'analyser directement les espèces capturées par la surface de capture au moyen de techniques histologiques classiques (coloration, immuno marquage) sur tissu frais, ce qui n'est pas réalisable sur un tissu prélevé par biopsie conventionnelle, pour lequel il est nécessaire au préalable de figer le tissu (par exemple par congélation, fixation et/ou inclusion en paraffine) afin de générer des coupes histologiques.

### Validations expérimentales

Un support de capture en silicium nanoporeux a été testé au moyen d'un essai ex vivo consistant à déposer 10 µl de liquide céphalo-rachidien humain directement sur la surface de capture.

Ensuite, la surface a été rincée deux fois à l'aide d'un tampon acide (acétate de sodium 100 mM, pH 4,0) pendant 1 minute. Ce rinçage permet d'éliminer les espèces n'ayant pas adhéré à la surface de capture et/ou des impuretés (résidus de tissu, sang, etc.). Ensuite, la surface a été rincée encore une fois dans l'eau, puis elle a été séchée à l'air libre. Une matrice organique (acide sinapinique pour l'analyse des protéines, ou CHCA (acide α-cyano-4-hydroxycinnamique) pour l'analyse des peptides) a été déposée sur la surface de capture, qui a ensuite été soumise à une analyse au moyen d'un spectromètre de masse SELDI du commerce (Biorad PCS 4000).

Les paramètres de lecture ont été réglés en fonction de l'échelle de masse des espèces à détecter. Les conditions optimales ont été déterminées manuellement sur quelques spots avant de lancer l'acquisition automatique sur 583 impacts laser répartis régulièrement pour chaque échantillon :
- pour les peptides (bas poids moléculaire), on a utilisé une intensité de 1000 nJ et une atténuation du signal de matrice à 500 Da,
- pour les protéines (haut poids moléculaire), on a utilisé une intensité de 2200 nJ et une atténuation du signal de matrice à 1000 Da.

La même analyse a été réalisée à titre comparatif et avec le même protocole en utilisant comme support de capture un porte-substrat du type normalement utilisé en spectrométrie de masse, à savoir une barrette métallique lisse sur laquelle est déposé un polymère, dont la référence est Biorad CM10.

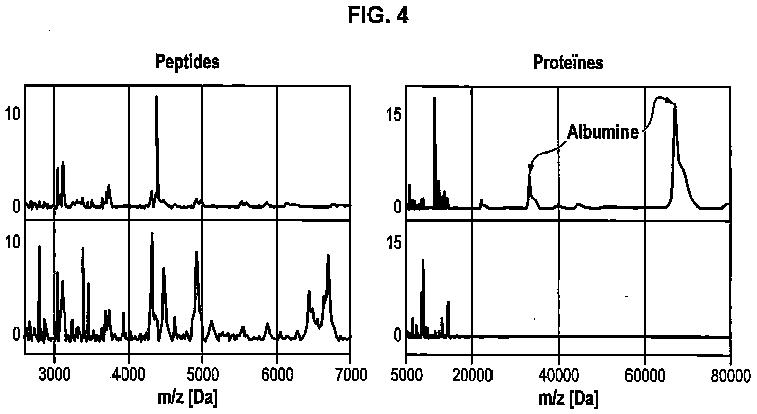

Les résultats sont représentés sur la figure 4, où les graphiques supérieurs ont été obtenus avec le porte-substrat lisse de référence et les graphiques inférieurs avec le support de capture en silicium nanoporeux.

Concernant les protéines (graphes de droite), on observe que les « petites » protéines sont fixées efficacement sur la surface de capture de silicium nanoporeux tandis que les « grandes » protéines, et notamment l'albumine sont sensiblement éliminées.

La différence entre les deux supports est encore plus nette dans le cas des peptides (graphes de gauche : ces derniers sont largement éliminés par rinçage dans le cas du support lisse alors que dans le cas du support de capture nanoporeux on observe un spectre très riche.

Un autre essai a été mis en oeuvre ex vivo pour caractériser la capacité du support de capture nanoporeux employé dans l'invention à fournir des informations différenciées en fonction de la zone de prélèvement sur un même organe.

A cet effet, le dispositif de prélèvement comprenant un support de capture en silicium nanoporeux a été employé pour réaliser une empreinte extemporanée d'un tissu de prostate sur une pièce d'exérèse fraîche.

A cet effet, le tissu frais a été apposé sur la surface de capture, puis la surface de capture a été rincée deux fois avec de l'éthanol 100% pendant 1 minute.

Ensuite, la surface de capture a été rincée rapidement dans de l'eau et a été laissée à sécher à l'air libre.

Une matrice organique (1 à 2 µl de CHCA) a été déposée sur la surface de capture, puis une analyse MALDI a été effectuée directement sur la surface de capture.

Le dispositif utilisé est un équipement MALDI TOF TOF Ultraflex Extreme de Bruker, les paramètres étant : énergie Laser comprise entre 60 et 70 %, atténuation du signal de matrice (Déflecteur) à 500 Da, en mode linéaire.

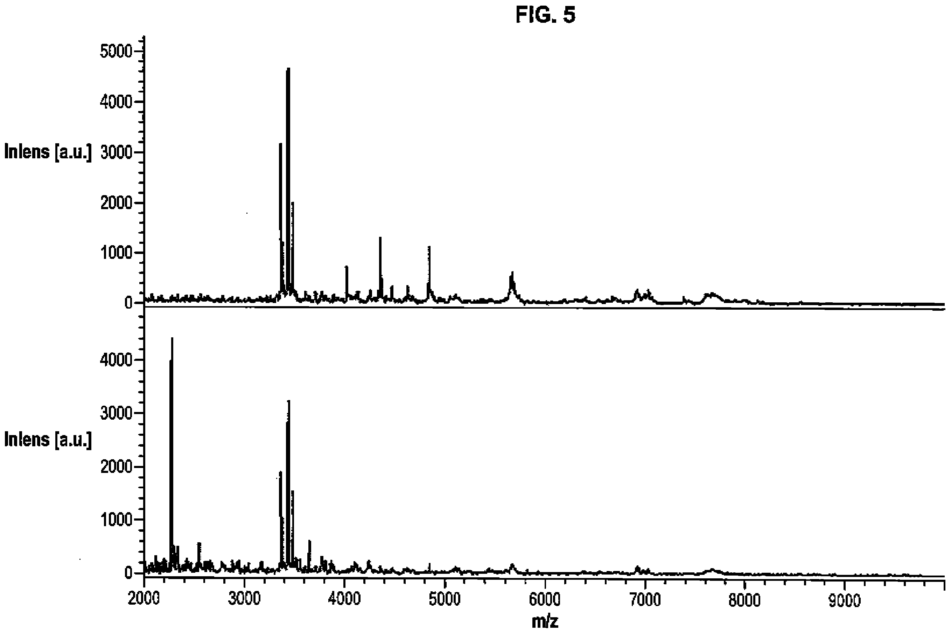

Les deux graphes de la figure 5 montrent le profilage protéique (intensité en fonction du rapport m/z) obtenu par spectrométrie MALDI pour deux zones différentes de la prostate.

On observe que les profils sont différents d'une zone à l'autre, ce qui montre que le support de capture permet de rendre compte des spécificités des tissus de deux zones d'un même organe.

Enfin, il va de soi que les exemples que l'on vient de donner ne sont que des illustrations particulières en aucun cas limitatives quant aux domaines d'application de l'invention, notamment en ce qui concerne les indications médicales et les espèces à analyser.

### REFERENCES

WO 2006/082344

## Revendications

1. Dispositif de prélèvement in vivo d'espèces biologiques, comprenant :
- un support de capture (1) en un matériau nanoporeux, présentant une surface (1a) de capture desdites espèces,
- une tige de préhension (3),
le dispositif de prélèvement étant **caractérisé en ce que** le support de capture (1) est porté par un plateau (2), et **en ce qu'**il comprend en outre une articulation (4) comprenant un premier membre (4a) couplé à la tige de préhension (3) et un second membre (4b) couplé de manière amovible au plateau (2) portant le support de capture (1), de manière à permettre de remplacer ledit plateau (2), de sorte que la surface (1a) de capture est sélectivement orientable par rapport à la tige de préhension (3), de façon à ajuster l'orientation du support de capture (1) par rapport à la tige de préhension (3).

2. Dispositif selon la revendication 1, **caractérisé en ce que** le premier (4a) et le second (4b) membres de l'articulation (4) sont séparables de sorte à détacher le support de capture (1) de la tige de préhension (3).

3. Dispositif selon l'une des revendications 1 ou 2, **caractérisé en ce que** le support de capture (1) est encastré dans le plateau (2), ledit plateau (2) présentant un rebord (2a) affleurant avec la surface (1a) de capture, de sorte que seule la surface (1a) de capture dudit support soit en contact avec des tissus lors du prélèvement in vivo.

4. Dispositif selon l'une des revendications 1 à 3, **caractérisé en ce que** le plateau (2) est en un matériau électriquement conducteur.

5. Dispositif selon l'une des revendications 1 à 4, **caractérisé en ce que** l'articulation (4) est une rotule.

6. Dispositif selon l'une des revendications 1 à 5, **caractérisé en ce que** le matériau du support de capture (1) est du silicium nanoporeux.

7. Dispositif selon l'une des revendications 1 à 6, **caractérisé en ce que** la surface (1a) de capture est inscrite dans un cercle de moins de 10 mm de diamètre.

8. Dispositif selon l'une des revendications 1 à 7, **caractérisé en ce qu'**il comprend en outre un dispositif d'orientation du support de capture, comprenant un câble s'étendant le long de la tige de préhension et couplé au second membre de l'articulation.

## Patentansprüche

1. Entnahmeverfahren in vivo von biologischen Arten, umfassend:
(i) einen Erfassungsträger (1) aus einem nanoporösen Material, der eine Erfassungsoberfläche (1a) der genannten Arten aufweist;
(ii) einen Greiferstift (3),
wobei die Greifervorrichtung **dadurch gekennzeichnet ist, dass** der Erfassungsträger (1) von einer Platte (2) getragen ist und dass sie darüber hinaus eine Artikulation (4), die ein erstes Glied (4a) umfasst, das an den Greiferstift (3) gekoppelt ist, und ein zweites Glied (4b), das abnehmbar an der Platte (2) gekoppelt ist, die den Erfassungsträger (1) trägt, derart umfasst, dass der Ersatz der genannten Platte (2) derart zugelassen wird, dass die Erfassungsfläche (1a) selektiv im Verhältnis zum Greiferstift (3) derart ausrichtbar ist, dass die Ausrichtung des Erfassungsträgers (1) im Verhältnis zum Greiferstift (3) angepasst wird.

2. Vorrichtung gemäß Anspruch 1, **dadurch gekennzeichnet, dass** das erste (4a) und das zweite (4b) Glied der Artikulation (4) derart trennbar sind, dass der Erfassungsträger (1) des Greiferstiftes (3) abgelöst wird.

3. Verfahren gemäß irgendeinem der Ansprüche 1 oder 2, **dadurch gekennzeichnet, dass** der Erfassungsträger (1) in der Platte (2) eingebaut ist, wobei die genannte Platte (2) einen Rand (2a) aufweist, der mit der Erfassungsoberfläche (1a) derart bündig abschließt, dass nur die Erfassungsoberfläche (1a) des genannten Trägers bei der Entnahme in vivo mit Geweben in Kontakt ist.

4. Verfahren gemäß irgendeinem der Ansprüche 1 bis 3, **dadurch gekennzeichnet, dass** die Platte (2) aus einem elektrisch leitenden Material ist.

5. Verfahren gemäß irgendeinem der Ansprüche 1 bis 4, **dadurch gekennzeichnet, dass** die Artikulation (4) ein Kugelgelenk ist.

6. Verfahren gemäß irgendeinem der Ansprüche 1 bis 5, **dadurch gekennzeichnet, dass** das Material des Erfassungsträgers (1) nanoporöses Silizium ist.

7. Verfahren gemäß irgendeinem der Ansprüche 1 bis 6, **dadurch gekennzeichnet, dass** die Erfassungsfläche (1a) einen Kreis von weniger als 10 mm Durchmesser bildet.

8. Verfahren gemäß irgendeinem der Ansprüche 1 bis 7, **dadurch gekennzeichnet, dass** sie darüber hinaus eine Ausrichtungsvorrichtung des Erfassungsträgers umfasst, umfassend ein Kabel, das sich entlang des Greiferstiftes erstreckt und mit dem zweiten Glied der Artikulation gekoppelt ist.

## Claims

1. A device for in vivo sampling of biological species, comprising:
- a capture support (1) in a nanoporous material, having a surface (1a) for the capture of said species,
- a grasping rod (3),
the sampling device being **characterized in that** the capture support (1) is borne by a plate (2), and **in that** it further comprises a joint (4) comprising a first member (4a) coupled with the grasping rod (3) and a second member (4b) removably coupled with the plate (2) bearing the capture support (1), so as to make it possible to replace said plate (2), so that the capture surface (1a) is selectively orientable relatively to the grasping rod (3), so as to adjust the orientation of the capture support (1) relatively to the grasping rod (3).

2. The device according to claim 1, **characterized in that** the first (4a) and the second (4b) members of the joint (4) may be separated so as to detach the capture support (1) from the grasping rod (3).

3. The device according to one of claims 1 or 2, **characterized in that** the capture support (1) is fitted into the plate (2), said plate (2) having a rim (2a) flush with the capture surface (1a), so that only the capture surface (1a) of said support is in contact with tissues during *in vivo* sampling.

4. The device according to one of claims 1 to 3, **characterized in that** the plate (2) is in an electrically conducting material.

5. The device according to one of claims 1 to 4, **characterized in that** the joint (4) is a ball joint.

6. The device according to one of claims 1 to 5, **characterized in that** the material of the capture support (1) is nanoporous silicon.

7. The device according to one of claims 1 to 6, **characterized in that** the capture surface (1a) is included in a circle with a diameter of less than 10 mm.

8. The device according to one of claims 1 to 7, **characterized in that** it further comprises a device for orienting the capture support, comprising a cable extending along the grasping rod and coupled with the second member of the joint.
